# EUROPEAN PATENT SPECIFICATION

(11) **EP 3 117 775 B1**
(45) Date of publication and mention of the grant of the patent: **14.08.2024**
(21) Application number: 15761360.5
(22) Date of filing: 23.02.2015
(51) Int. Cl.: A61B 8/12, A61B 8/14, A61B 10/00

(54) **CONTROL DEVICE AND DIAGNOSIS SYSTEM FOR SAME**
STEUERUNGSVORRICHTUNG SOWIE DIAGNOSESYSTEM DAFÜR
DISPOSITIF DE COMMANDE ET SYSTÈME DE DIAGNOSTIC ASSOCIÉ

(30) Priority: 12.03.2014 JP 2014049290
(43) Date of publication of application: 18.01.2017
(73) Proprietor: Terumo Kabushiki Kaisha, Tokyo 151-0072 (JP)
(72) Inventor: MORI, Isao, Ashigarakami-gun Kanagawa 259-0151 (JP)
(74) Representative: Casalonga
(86) International application number: PCT/JP2015/000876
(87) International publication number: WO 2015/136854

(56) References cited:
- WO-A1-2013/145711
- JP-A- 2006 192 059
- JP-A- 2009 125 394
- JP-A- 2009 125 394
- JP-A- 2010 011 964
- JP-A- 2011 072 596
- US-A1- 2007 066 890
- LI JIAWEN ET AL: "Back-to-back optical coherence tomography-ultrasound probe for co-registered three-dimensional intravascular imaging with real-time display", PROGRESS IN BIOMEDICAL OPTICS AND IMAGING, SPIE - INTERNATIONAL SOCIETY FOR OPTICAL ENGINEERING, BELLINGHAM, WA, US, vol. 8934, 4 March 2014 (2014-03-04), pages 893428 - 893428, XP060033546, ISSN: 1605-7422, ISBN: 978-1-5106-0027-0, DOI: 10.1117/12.2038270
- LI XIANG ET AL: "Integrated IVUS-OCT Imaging for Atherosclerotic Plaque Characterization", IEEE JOURNAL OF SELECTED TOPICS IN QUANTUM ELECTRONICS, IEEE SERVICE CENTER, PISCATAWAY, NJ, US, vol. 20, no. 2, 1 March 2014 (2014-03-01), pages 7100108, XP011527021, ISSN: 1077-260X, [retrieved on 20130918], DOI: 10.1109/JSTQE.2013.2274724

## Description

### Technical Field

The present invention relates to a control device and a diagnosis system. In particular, the technical field relates to a control device according to the preamble of claim 1, such as it is e.g. known from US2007/66890A1.

### Background Art

Conventionally, diagnostic imaging apparatuses have been in wide use for a diagnosis of arteriosclerosis and a preoperative diagnosis or a postoperative result check in intravascular treatment with a high performance catheter such as a balloon catheter or a stent. For example, as representative diagnostic imaging apparatuses, intravascular ultrasound (IVUS) and optical coherent tomography/optical frequency domain imaging (OCT/OFDI) have been developed.

These diagnostic imaging apparatuses have also been used to develop techniques that assist in a diagnosis by physicians . Patent Document 1 discloses a technique for displaying a vascular axial sectional image (a vertical sectional image, a sectional image in a section parallel to a vascular axis, or a sectional image in a plane passing the vascular axis) in a blood vessel, obtained using a diagnostic imaging apparatus . In this technique, when a user designates a desired position on the vascular axial sectional image, a cross-sectional image (a sectional image in a direction crossing the vascular axis or a sectional image in a plane perpendicular to the vascular axis) corresponding to the designated position is again displayed. In addition, when the user designates the desired position on the vascular axial sectional image, a signal transceiver can be automatically moved to the designated position.

### Related Art

### Patent Document

Patent Document 1: JP-A-2011-072597

### Disclosure of the Invention

### Problem that the Invention is to solve

Different kinds of diagnostic imaging apparatuses have different characteristics. For example, IVUS performs radial scanning by emitting an ultrasound wave into a blood vessel while rotating a transceiver consisting of an ultrasound transducer under a state where an ultrasound probe part containing the transceiver is inserted in the blood vessel, and receiving a reflected wave from a living body. A vascular sectional image is extracted based on the strength of an ultrasound echo signal generated by subjecting the reflected wave obtained thus to processes such as an amplification, detection and so on. In general, IVUS can acquire an image of a deeper portion of the blood vessel than OFDI.

In addition, OCT performs a radial scanning by emitting measurement light into a blood vessel while rotating a transceiver with its distal end to which an optical lens and an optical mirror are attached, under a state where an optical probe part containing the transceiver and an optical fiber is inserted in the blood vessel, and receiving reflected light from living body tissues. A vascular sectional image based on interference light is extracted by causing the reflected wave obtained thus and reference light separated from the measurement light to interfere with each other. OFDI has basically the same configuration as OCT but is characterized in that the former successively emits light with different wavelengths. In addition, when the strength of reflected light at points in the depth direction of living body tissues is obtained through frequency analysis of interference light, there is no need for a mechanism for varying an optical path length of reference light. In general, OCT/OFDI can obtain images having a higher resolution than IVUS.

Although correct diagnosis by physicians may be supported by comparing these images having different characteristics, such a technique has not yet sufficiently been developed.

A method for assisting in a diagnosis using images obtained by a plurality of signal transceivers is also discussed in the disclosure.

### Means for solving the Problem

To solve the above problems, the present invention provides a control device according to independent claim 1. The dependent claims relate to advantageous embodiments.

### Effects of the Invention

The control device of the present invention may be used in a method for assisting in a diagnosis using images obtained by a plurality of signal transceivers.

These and other features and advantages of the present invention will be apparent from the following description in conjunction with the accompanying drawings. Throughout the accompanying drawings, the same or similar configurations are denoted by the same reference numerals.

### Brief Description of the Drawings

The accompanying drawings are included in the present application and constitute a part thereof. The accompanying drawings illustrate exemplary embodiments of the present invention and are used to explain the principle of the present invention along with the following description.
FIG. 1 is a view illustrating a diagnostic imaging system according to one embodiment;
FIG. 2A is a view illustrating a probe part of the diagnostic imaging system according to one embodiment;
FIG. 2B is a view illustrating a probe part of the diagnostic imaging system according to one embodiment;
FIG. 3 is a view illustrating the functional configuration of a control device according to Embodiment 1;
FIG. 4A is a view illustrating a radial scanning according to one embodiment;
FIG. 4B is a view illustrating a radial scanning according to one embodiment;
FIG. 5 is a view illustrating a display screen in Embodiment 1;
FIG. 6 is a flow chart of a process in Embodiment 1;
FIG. 7 is a flow chart of a process in Embodiment 2;
FIG. 8 is a view illustrating the configuration of a computer used in Embodiment 3;
FIG. 9 is a view illustrating a relationship between lapse time and motion of a probe part in Embodiment 1; and
FIG. 10 is a view illustrating a relationship between lapse time and motion of a probe part in Embodiment 2.

### Embodiments for Carrying Out the Invention

Hereinafter, exemplary embodiments of the present invention will be described with reference to the accompanying drawings.

### [Embodiment 1]

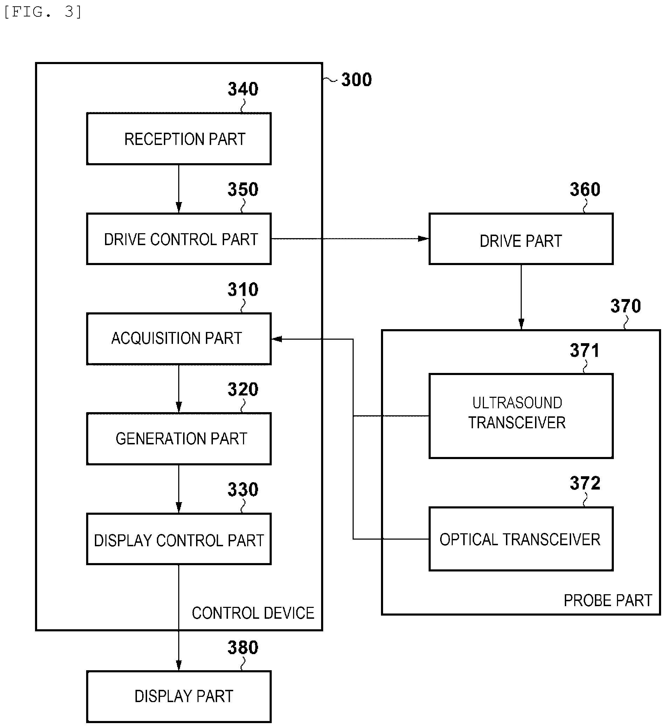

FIG. 3 illustrates a control device 300 according to Embodiment 1. The control device 300 includes an acquisition part 310, a generation part 320, a display control part 330, a reception part 340, and a drive control part 350.

The acquisition part 310 acquires a signal measured using a first signal transceiver on a probe 370 during first driving and information indicating a position of the first signal transceiver at a point of time when this signal is obtained. In addition, the acquisition part 310 acquires a signal measured using a second signal transceiver on the probe 370 during second driving and information indicating a position of the second signal transceiver at a point of time when this signal is obtained. In this embodiment, the first signal transceiver is an ultrasound transceiver 371 (ultrasound sensor) and the second signal transceiver is an optical transceiver 372 (optical fiber and lens). However, the types of the first and second signal transceivers are not limited thereto. In addition, as described above, in a case of OCT/OFDI, the probe 370 has only an optical fiber and a lens, but does not have an element corresponding to an optical sensor (photodetector). In this case, the element corresponding to the optical sensor exists in the control device 300.

The generation part 320 generates an ultrasound cross-sectional image and an ultrasound vascular axial sectional image of a blood vessel based on a signal from the ultrasound transceiver 371 (a cross-sectional image and a vascular axial sectional image generated by the ultrasound transceiver 371). In addition, the generation part 320 may generate an optical cross-sectional image and an optical vascular axial sectional image of a blood vessel based on a signal from the optical transceiver 372 (a cross-sectional image and a vascular axial sectional image generated by the optical transceiver 372).

The display control part 330 causes a display part 380 to simultaneously display a vascular image at a first position in the blood vessel, which is obtained by the ultrasound transceiver 371, and a vascular image at the first position in the blood vessel, which is obtained by the optical transceiver 372. At this time, the display control part 330 refers to the information indicating the positions of the ultrasound transceiver 371 and the optical transceiver 372, which is acquired by the acquisition part 310. The display part 380 is not particularly limited as long as it can display information. For example, the display part 380 may be an LCD monitor 113 to be described later.

The reception part 340 receives a designation of a measurement range by the optical transceiver 372 during or after driving for measurement by the ultrasound transceiver 371. Detailed configuration of the reception part 340 is not particularly limited. For example, the reception part 340 may be an operation panel 112 to be described later.

The drive control part 350 controls driving of the probe 370 by a drive part 360 driving the probe 370 inserted in the blood vessel. Specifically, the drive control part 350 sends a control signal to the drive part 360, which causes the drive part 360 to execute the first driving for measurement using the ultrasound transceiver 371 on the probe 370 and the second driving for measurement using the optical transceiver 372 on the probe 370.

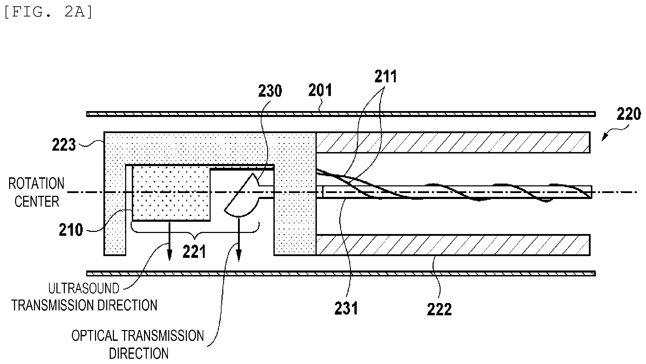

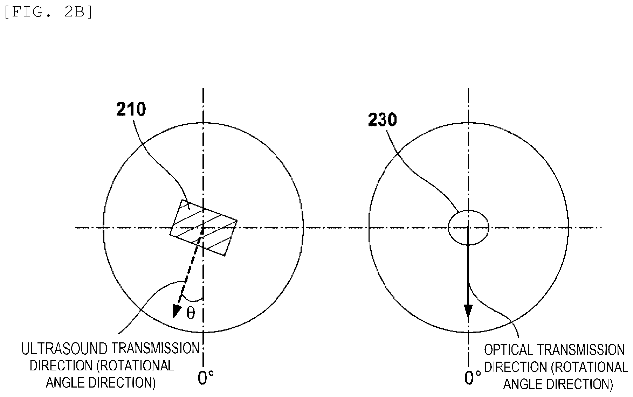

Hereinafter, the function and configuration of the control device 300 will be described in more detail. The following description will be given under the presumption that the control device 300 is contained in a diagnostic imaging system 100 having both an IVUS function and an OCT function. That is, in the following description, the ultrasound transceiver 371 corresponds to an ultrasound transceiver 210 illustrated in FIGS. 2A and 2B, and the optical transceiver 372 corresponds to an optical transceiver 230 illustrated in FIGS. 2A and 2B. It should be, however, noted that a method for generating a vascular image and a signal transceiver used for the method are not limited thereto but any types of signal transceivers may be used.

### <Method for Acquiring Blood Vessel Image>

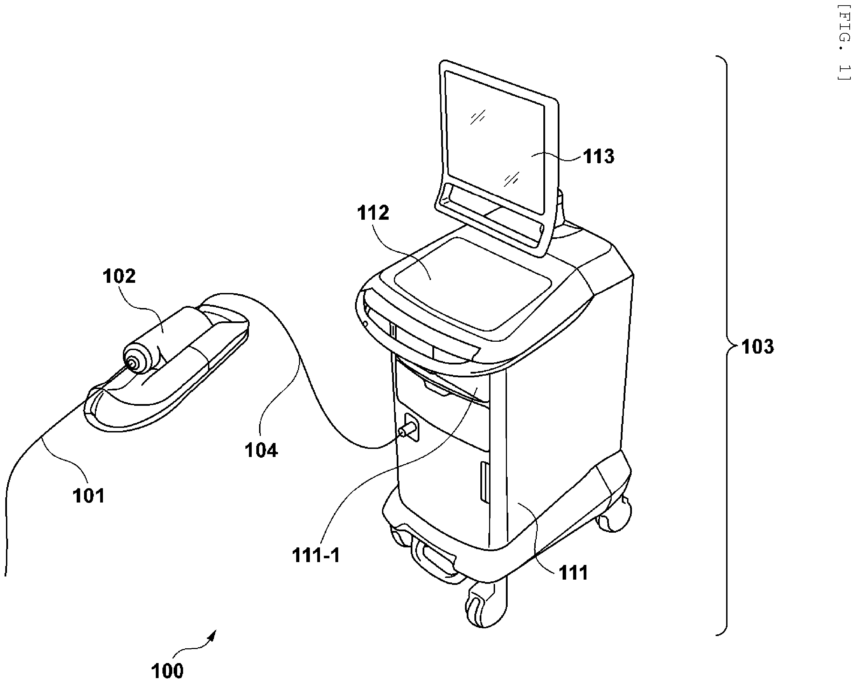

A method for acquiring a vascular image will be briefly described first. FIG. 1 is a view illustrating the external configuration of a diagnostic imaging system (having both an IVUS function and an OCT function) 100 according to one embodiment of the present invention. As illustrated in FIG. 1, the diagnostic imaging system 100 includes a probe part 101, a scanner/pullback part 102, and an operation control device 103. The scanner/pullback part 102 and the operation control device 103 are connected by a signal line 104 for signal communication therebetween.

The probe part 101 contains an imaging core 220 to be directly inserted in the blood vessel. The imaging core 220 includes the ultrasound transceiver 210 which transmits an ultrasound wave based on a pulse signal into the blood vessel and receives a reflected wave from the interior of the blood vessel. In addition, the imaging core 220 includes the optical transceiver 230 which continuously transmits light (measurement light) into the blood vessel and receives reflected light from the interior of the blood vessel. In the diagnostic imaging system 100, the imaging core 220 is used to measure the state of the interior of the blood vessel.

The scanner/pullback part 102 drives the probe part 101 inserted in the blood vessel. Specifically, the probe part 101 is detachably connected to the scanner/pullback part 102 and a motor contained in the scanner/pullback part 102 is driven to specify intravascular axial and rotational operations of the imaging core 220 inserted in the probe part 101. In addition, the scanner/pullback part 102 acquires the reflected wave received in the ultrasound transceiver and the reflected light received in the optical transceiver, and transmits the reflected wave and light to the operation control device 103.

For measurement, the operation control device 103 has a function of inputting a variety of setting values, and a function of processing data obtained by measurement in order to display a sectional image (a cross-sectional image and a vascular axial sectional image) in the blood vessel.

In the operation control device 103, reference numeral 111 denotes a body control part which generates ultrasound data based on the reflected wave obtained by measurement and generates an ultrasound sectional image by processing line data generated based on the ultrasound data. In addition, the body control part 111 generates interference light data by causing the reflected wave, which is obtained by measurement and reference light, which is obtained by separating light from a light source, to interfere with each other, and generates an optical sectional image by processing line data generated based on the interference light data.

Reference numeral 111-1 denotes a printer/DVD recorder which prints or stores a result of the processing in the body control part 111 as data. Reference numeral 112 denotes an operation panel through which a user inputs a variety of setting values and instructions. Reference numeral 113 denotes an LCD monitor as a display device which displays a sectional image generated in the body control part 111.

Next, the overall configuration of the probe part 101 and the sectional configuration of a distal portion thereof will be described with reference to FIG. 2A. The probe part 101 is constituted by a long catheter sheath 201 to be inserted in the blood vessel, and a connector which is not inserted in the blood vessel for user's operation and is disposed at hand side of the user.

The imaging core 220 is inserted inside a lumen of the catheter sheath 201 over substantially the full length of the catheter sheath 201. The imaging core 220 includes a housing 223 and a drive shaft 222.

The drive shaft 222 of a coil shape transmits a rotational driving force for rotating the housing 223. The drive shaft 222 is constituted by a flexible multiplex multi-layered tightly-wound coil formed of, e.g., a metal line such as stainless steel, which allows a transceiver unit 221 to be rotated and axially actuated with respect to the catheter sheath 201 and has the characteristic that rotation can be transmitted properly. In addition, an electrical signal cable 211 and an optical fiber cable 231 (optical fiber cable of a single mode) are disposed inside the drive shaft 222. The electrical signal cable is connected with the ultrasound transceiver 210 and the optical fiber cable 231 is connected to the optical transceiver 230. The electrical signal cable 211 is wound spirally on the optical fiber cable 231.

The housing 223 has a shape of a short cylindrical metal pipe having a partial notched portion and formed by cutting a metal ingot or by means of metal-power injection molding (MIM) or the like. The transceiver unit 221 including the ultrasound transceiver 210 and the optical transceiver 230 is placed in the housing 223. The ultrasound transceiver 210 and the optical transceiver 230 are disposed axially on the rotational center axis (indicated by a dashed line in FIG. 2A) of the drive shaft 222. The ultrasound transceiver 210 is disposed at the distal end side of the probe part 101 and the optical transceiver 230 is disposed at the proximal end side of the probe part 230.

In addition, the ultrasound transceiver 210 and the optical transceiver 230 are mounted inside the housing 223 in such a manner that the ultrasound transmission direction (elevational angle direction) of the ultrasound transceiver 210 and the optical transmission direction (elevational angle direction) of the optical transceiver 230 become about 90° with respect to the axial direction of the drive shaft 222. In addition, it is advantageous that each of the transmission directions is slightly deviated from 90° so as not to receive a reflection at the inner surface of the lumen of the catheter sheath 201. In addition, as illustrated in FIG. 2B, the ultrasound transceiver 210 and the optical transceiver 230 are disposed in such a manner that the ultrasound transmission direction (rotational angle direction (also referred to as an azimuthal angle direction)) of the ultrasound transceiver 210 and the optical transmission direction (rotational angle direction) of the optical transceiver 230 are deviated by θ° from each other.

Next, a vascular image generating method using the ultrasound transceiver 210 will be described. The ultrasound transceiver 210 emits an ultrasound wave to a living body based on a pulse wave transmitted from the body control part 111, receives a reflected wave (echo) from the living body, and transmits the received echo to the body control part 111. At this time, the scanner/pullback part 102 is driven to rotate the imaging core 220. A rotational angle at this time is detected by an encoder contained in the scanner/pullback part 102. In addition, the scanner/pullback part 102 specifies the axial operation of the imaging core 220. At this time, an axial position of the imaging core 220 is detected by the scanner/pullback part 102.

An ultrasound signal received by the ultrasound transceiver 210 is detected in the body control unit 111. Thereafter, the body control part 111 samples the obtained ultrasound signal to generate digital data (ultrasound data) of one line indicating information of depth direction along the direction of the ultrasound transceiver 210. Hereinafter, the data obtained thus will be referred to as line data.

Next, a vascular image generating method using the optical transceiver 230 will be described. The optical transceiver 230 emits light (measurement light), which is transmitted from the body control part 111, to a living body in the blood vessel. At this time, as described above, the imaging core 220 is driven to be rotated by the scanner/pullback part 102 and is axially operated. Some of reflected light scattered in the surface or interior of the living body is received by the optical transceiver 230 and is transmitted to the body control part 111 through a reverse optical path. The reflected light from the optical transceiver 230, which is transmitted thus, is mixed with the reference light and is received and converted into an electrical signal by a photodiode contained in the body control part 111. Thus, an interference light signal about interference light obtained by mixing the reflected light and the reference light can be obtained.

The body control part 111 generates digital data (interference light data) of one line by sampling the interference light signal. Thereafter, the body control part 111 generates data of depth direction along the direction of the optical transceiver 230 by resolving a frequency of the generated interference light data of one line by means of fast Fourier transform (FFT) . Hereinafter, the data obtained thus will be referred to as line data.

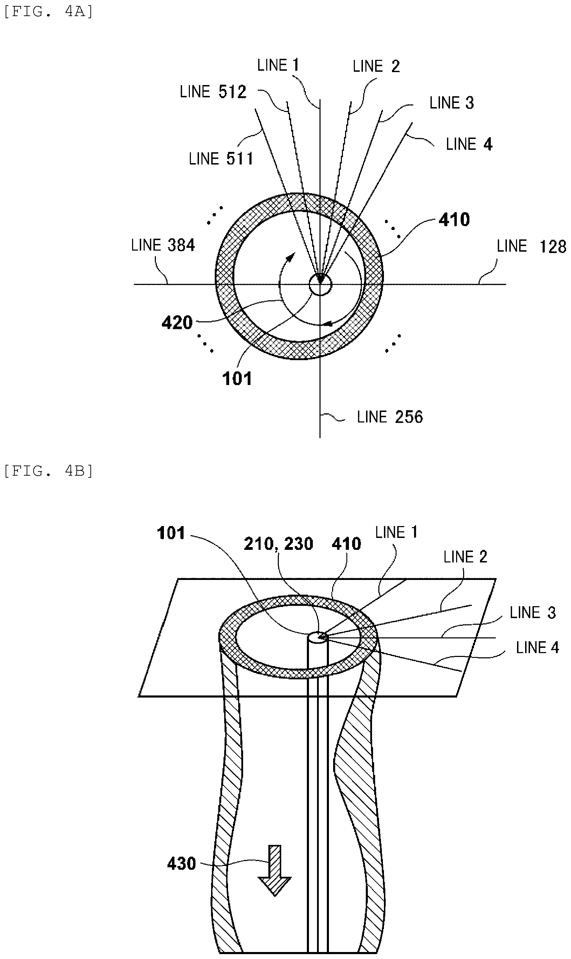

During the measurement using the ultrasound transceiver 210 or the optical transceiver 230, while the probe part 101 is rotated as described above, the ultrasound transceiver 210 and the optical transceiver 230 attached to the distal end of the probe part 101 are also rotated in a direction indicated by an arrow 420. Each of the transceivers 210 and 230 performs transmission/reception of the ultrasound wave or measurement light at the respective rotational angle. In FIG. 4A, lines 1, 2, ..., 512 denote transmission directions of the ultrasound wave or measurement light at the respective rotational angle. In the embodiment illustrated in FIG. 4A, while the transceivers 210 and 230 are being rotated by 360° in a predetermined vascular section 410, transmission/reception of the ultrasound wave or measurement light is intermittently performed 512 times. In the meantime, the number of times of transmission/reception (the number of measurement lines) of the ultrasound wave or measurement light during the 360° rotation is not particularly limited thereto but may be set at random. In addition, the number of times of transmission/reception of the ultrasound wave may be different from the number of times of transmission/reception of the measurement light. For example, the number of times of transmission/reception of the ultrasound wave may be 2048 and the number of times of transmission/reception of the measurement light may be 512 . Such transmission/reception of the ultrasound wave or measurement light is performed while the transceivers 210 and 230 are being travelled inside the blood vessel in a direction indicated by an arrow 430, as illustrated in FIG. 4B. Hereinafter, a series of operations in which the ultrasound transceiver 210 or the optical transceiver 230 performs a scanning while moving and rotating in the axial direction will be referred to as a radial scanning.

The obtained line data are conserved in association with information indicating the position of the ultrasound transceiver 210 or the optical transceiver 230. In one embodiment, a pulse signal output every time the scanner/pullback part 102 drives the probe 101 linearly by a predetermined amount is counted by a movement amount detector contained in the scanner/pullback part 102. The line data are conserved in association with this count value.

In this embodiment, the number of pulse signals output when the scanner/pullback part 102 pulls out the probe part 101 is added to the count value, whereas the number of pulse signals output when the scanner/pullback part 102 pushes out the probe part 101 is subtracted from the count value. Typically, the line data are generated sufficiently quickly after a signal is acquired from the ultrasound transceiver 210 or the optical transceiver 230. Therefore, the count value obtained thus reflects the position of the ultrasound transceiver 210 or the optical transceiver 230 when a signal is acquired from the ultrasound transceiver 210 or the optical transceiver 230.

In addition, the obtained line data are also associated with information indicating the measurement direction of the ultrasound transceiver 210 or the optical transceiver 230. In one embodiment, information indicating the current measurement line, which is output from the scanner/pullback part 102, is conserved in association with the line data. In the example of FIG. 4A, any of Nos. 1 to 512 is conserved as the number of the measurement line corresponding to the line data.

As described above, the generation part 320 constructs a sectional image by using the line data stored in association with the count value. For example, the generation part 320 may generate a cross-sectional image corresponding to each position by performing the Rθ transformation after performing a variety of processes (line addition averaging process, filtering process and so on). The cross-sectional image generated thus is also conserved in association with the count value. That is, for each cross-sectional image, the position of the ultrasound transceiver 210 or the optical transceiver 230 when a signal is acquired, is specified.

Further, the generation part 320 may generate a vascular axial sectional image by using the line data stored in association with the count value. In one embodiment, for each of line data (including line data from line 1 to line 512) in each of count values, the generation part 320 extracts predetermined line data (line data of two lines corresponding to any coordinate axis passing a sectional image center coordinate when the sectional image is constructed (i. e. , line data in mutual 180° relationship)). Subsequently, the generation part 320 arranges line data two by two lines, which are extracted from each line data, at an axial position corresponding to the count value associated with each line data. Thus, a vascular axial sectional image having a horizontal axis representing the count value and a vertical axis representing the line data (specifically, a pixel value of the line data) is constructed.

The generation part 320 may also subject the cross-sectional image or the vascular axial sectional image to a variety of processes. The cross-sectional image or the vascular axial sectional image obtained thus is displayed on the display part 380 by the display control part 330. In one embodiment, the display control part 330 may display the cross-sectional image or the vascular axial sectional image, which is sequentially generated or updated during the measurement by the ultrasound transceiver 210 or the optical transceiver 230, on the display part 380 in real time.

### <Process in Embodiment>

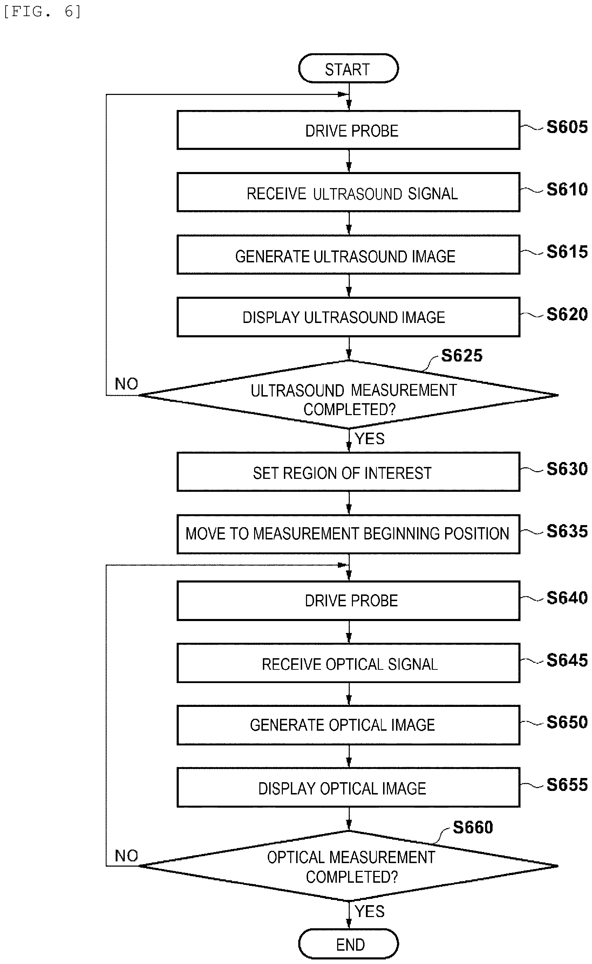

Hereinafter, a control method in Embodiment 1 will be described with reference to a flow chart of FIG. 6. At Steps S605 to S625, an intravascular measurement using the ultrasound transceiver 371 is performed. In this embodiment, measurement is continuously performed over a set vascular length-wise distance.

More specifically, at Step S605, the drive control part 350 controls the drive part 360 to perform the intravascular measurement using the ultrasound transceiver 371. According to the control of the drive control part 350, the scanner/pullback part 102 constituting the drive part 360 controls the linear driving and rotational driving of the probe part 101 so as to perform intravascular radial scanning using the ultrasound transceiver 371. In this embodiment, the drive control part 350 controls the drive part 360 to perform the measurement at a set scanning speed over the set vascular length-wise distance.

At Step S610, the acquisition part 310 sequentially acquires an ultrasound signal obtained by the ultrasound transceiver 371 while performing the radial scanning using the ultrasound transceiver 371. At this time, the acquisition part 310 additionally acquires a signal indicating a position of the ultrasound transceiver 371 when the ultrasound signal is acquired. The signal indicating this position may be a count value indicating a linear movement amount of the scanner/pullback part 102, as described above,

At Step S615, the generation part 320 generates an ultrasound image based on the ultrasound signal acquired at Step S610. Specifically, the generation part 320 may generate an ultrasound cross-sectional image and an ultrasound vascular axial sectional image according to the above-described method. While the ultrasound transceiver 371 is performing the scanning, the process of Step S615 may be performed. For example, the generation part 320 may use the ultrasound signal, which is acquired when the ultrasound transceiver 371 is at a particular vascular position, to generate a vascular cross-sectional image at this vascular position. In addition, the generation part 320 may use the ultrasound signal, which is acquired until the ultrasound transceiver 371 arrives at the current vascular position after the ultrasound transceiver 371 begins to perform the scanning, to generate a vascular axial sectional image from the scanning beginning position to the current vascular position.

At Step S620, the display control part 330 displays the vascular image, which is generated at Step S615, on the display part 380. Specifically, the cross-sectional image at the current position of the ultrasound transceiver 371 and the vascular axial sectional image at the position at which the ultrasound transceiver 371 has performed the measurement up to now are displayed on the display part 380.

At Step S625, the drive control part 350 determines whether or not the measurement over the set vascular length-wise distance has been completed. When it is determined that the measurement has been completed, the process proceeds to Step S630. When it is determined that the measurement has not been completed, the process returns to Step S605 in which the radial scanning using the ultrasound transceiver 371 continues to be performed.

At Step S630, the reception part 340 receives a designation of a measurement range. For a measurement range designated by a user, re-measurement using the optical transceiver 372 is performed, as described later. Hereinafter, the measurement range designated at Step S630 will be referred to as a region of interest (ROI).

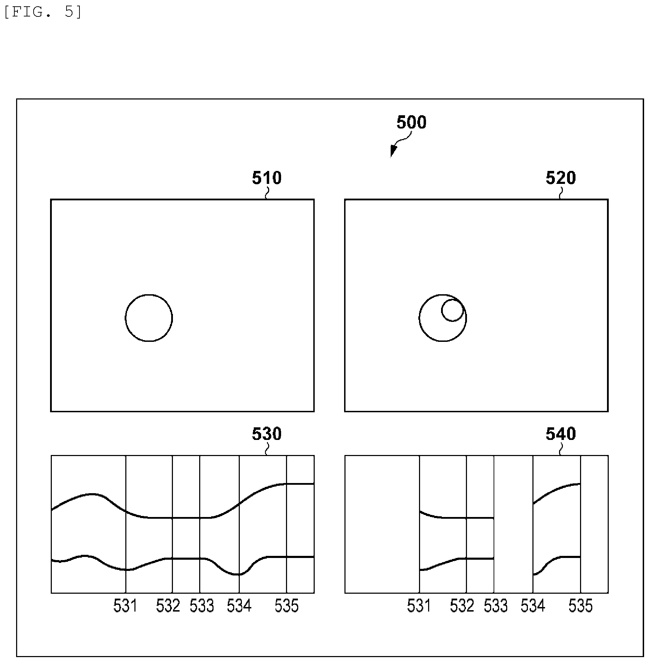

In one embodiment, the re-measurement using the optical transceiver 372 is performed for a portion of the measurement range using the ultrasound transceiver 371. In this embodiment, a user designates a measurement range while viewing the vascular axial sectional image which is being displayed on the display part 380 and is based on a signal obtained by the ultrasound transceiver 371. For example, when the user designates two points on the vascular axial sectional image by manipulation through the operation panel 112, a range sandwiched between the two points specified in the vascular length direction may be set as a region of interest. For example, as illustrated in FIG. 5, when an ultrasound vascular axial sectional image 530 is displayed on the display part 380, the user may designate a beginning position 531/an ending position 533 of measurement and a beginning position 534/an ending position 535 of measurement. In this manner, at Step S630, two or more regions of interest may be set. In this case, a process of Steps S635 to S660 to be described later is repeated for each region of interest.

However, a method for setting a region of interest is not limited to this method. For example, the user may designate one point on the vascular axial sectional view, in which case a predetermined range including the designated point is set as a region of interest. In one example, when the user designates a beginning position of measurement, a position separated by a predetermined distance from the designated point is used as an ending position of measurement. In addition, the user may designate a desired cross-sectional image while viewing a cross-sectional image, in which case a predetermined range including a measurement position of the designated cross-sectional image is set as a region of interest. In addition, it is not essential for the user to designate a measurement range. For example, the control device 300 may automatically set a region of interest based on a cross-sectional image or vascular axial sectional image obtained based on an ultrasound signal or ultrasound signal.

At Step S635, the drive control part 350 controls the drive part 360 to drive the probe 370 so as to move the optical transceiver 372 to a measurement beginning position. As described above, in this embodiment, a count value is associated with the amount of pulling-out of the probe part 101 in each cross-sectional image. In addition, a count value is associated with a horizontal axis in a vascular axial sectional image. Therefore, the drive control part 350 can know a count value corresponding to the measurement beginning position. In one embodiment, the probe 370 is moved by the drive part 360 such that the current count value becomes a count value corresponding to the measurement beginning position. However, in consideration of time required to stabilize an operation speed of the radial scanning, the probe 370 may be driven to move the optical transceiver 372 up to a position beyond the measurement beginning position.

However, strictly speaking, the vascular length-wise position of the ultrasound transceiver 210 is different from that of the optical transceiver 230. Therefore, in another embodiment, the probe 370 is moved to obtain a count value different by a predetermined value from the count value corresponding to the measurement beginning position. For example, when the probe part 101 illustrated in FIG. 2A is used, the probe 370 is moved to obtain a count value which is smaller by a predetermined value reflecting a distance between the ultrasound transceiver 371 and the optical transceiver 372 than the count value corresponding to the measurement beginning position. Typically, the measurement is performed while pulling out the probe. Therefore, a position farthest from an insertion position of the probe in a region of interest becomes a measurement beginning position and a position nearest to the insertion position of the probe in the region of interest becomes a measurement ending position.

At Steps S640 to S655, the optical transceiver 372 performs measurement for a designated region of interest. As is known in the art, before taking an optical image, a flush operation of releasing physiological saline, lactate Ringer's solution, a contrast agent or the like from a guide catheter (not illustrated) to remove the blood in a blood vessel of a portion to be imaged is performed.

Specifically, at Step S640, the drive control part 350 controls the drive part 360 to measure the interior of the blood vessel by means of the optical transceiver 372. A method of controlling the drive part 360 corresponds to Step S605. However, typically, the measurement by the optical transceiver 372 is performed for a region of interest which is narrower than a range of measurement by the ultrasound transceiver 371.

At Step S645, the acquisition part 310 sequentially acquires an optical signal obtained by the optical transceiver 372 while performing the radial scanning using the optical transceiver 372. At this time, like Step S610, the acquisition part 310 additionally acquires a signal indicating a position of the optical transceiver 372 when the optical signal is acquired. At Step S650, the generation part 320 generates an optical image based on the optical signal acquired at Step S645 . Specifically, the generation part 320 may generate a cross-sectional image and a vascular axial sectional image according to the above-described method, as in Step S615.

At Step S655, the display control part 330 displays the vascular image, which is generated at Step S650, on the display part 380. In this embodiment, a vascular image at a first position in the blood vessel, which is obtained by the ultrasound transceiver 371, and a vascular image at the first position in the blood vessel, which is obtained by the optical transceiver 372, are simultaneously displayed on the display part 380. Here, the displayed optical cross-sectional image and ultrasound cross-sectional image are cross-sectional images at the same position in the blood vessel.

As described above, in this embodiment, a count value is associated with the amount of pulling-out of the probe part 101 in each cross-sectional image. In one embodiment, an optical cross-sectional image and an ultrasound cross-sectional image, which are associated with the same count value, are simultaneously displayed. However, strictly speaking, the vascular length-wise position of the ultrasound transceiver 210 is different from that of the optical transceiver 230. Therefore, in another embodiment, a combination of an optical cross-sectional image and an ultrasound cross-sectional image, which are selected such that their count values are different by a predetermined value reflecting a distance between the ultrasound transceiver 371 and the optical transceiver 372 from each other, is simultaneously displayed on the display part 380. For example, when the probe part 101 illustrated in FIG. 2A is used, a combination of an ultrasound cross-sectional image associated with a certain count value and an optical cross-sectional image associated with a count value smaller by a predetermined value than the certain count value is displayed.

In this embodiment, when the optical transceiver 230 acquires an optical signal, a sequentially generated optical cross-sectional image is displayed on the display part 380. Therefore, an ultrasound cross-sectional image displayed at the same time is also changed every moment.

In one embodiment, the ultrasound cross-sectional image and the optical cross-sectional image are displayed such that a vascular inner wall located in the same measurement direction is displayed in the same direction when viewed from the position of the transceivers 371 and 372 on the display part 380. In other words, rotational angles of the ultrasound cross-sectional image and the optical cross-sectional image are adjusted such that their angular directions are aligned. As described above, the cross-sectional image is generated from the line data with which the information indicating the current measurement line is associated. In one embodiment, the generation part 320 generates an ultrasound cross-sectional image and an optical cross-sectional image such that the vascular inner wall on a predetermined measurement line (e.g. , line 1) is displayed in an upper side. However, it is illustrated in FIG. 2B that the ultrasound transmission direction of the ultrasound transceiver 210 and the optical transmission direction of the optical transceiver 230 are deviated by θ° from each other . However, this deviation between these transmission directions is not reflected in the information indicating the current measurement line output from the scanner/pullback part 102. Therefore, in another embodiment, while the ultrasound cross-sectional image is displayed such that a measurement line of a predetermined number is in an upper side, the optical cross-sectional image is displayed such that a measurement line of a number different by a numerical value reflecting the above-mentioned angle θ from the predetermined number is in the upper side. In addition, when the number of measurement lines for the measurement using the ultrasound transceiver 210 is different from the number of measurement lines for the measurement using the optical transceiver 230, numbers of measurement lines indicating the same angular direction are determined in consideration of such a difference in the number of measurement lines.

At Step S660, the drive control part 350 determines whether or not the measurement over a designated region of interest has been completed. When it is determined that the measurement has not been completed, the process returns to Step S640 in which the radial scanning using the optical transceiver 372 continues to be performed. When it is determined that the measurement has been completed, the process is ended.

FIG. 5 illustrates an example of a display screen 500 of the display part 380 when the process illustrated in FIG. 6 is ended. An ultrasound cross-sectional image 510, an ultrasound vascular axial sectional image 530, an optical cross-sectional image 520 and an optical vascular axial sectional image 540 are displayed on the display screen 500. The ultrasound cross-sectional image 510 at a vascular position 532 and the optical cross-sectional image 520 at the same vascular position 532 are illustrated in FIG. 5. In this manner, an optical image and an ultrasound image at the same intravascular position are simultaneously displayed on the display part 380. For example, when a user designates a desired vascular position 532 on the ultrasound vascular axial sectional image 530, the ultrasound cross-sectional image 510 at the vascular position 532 is displayed and the optical cross-sectional image 520 at the same vascular position 532 is also automatically displayed. The user may designate a vascular position on the optical vascular axial sectional image 540.

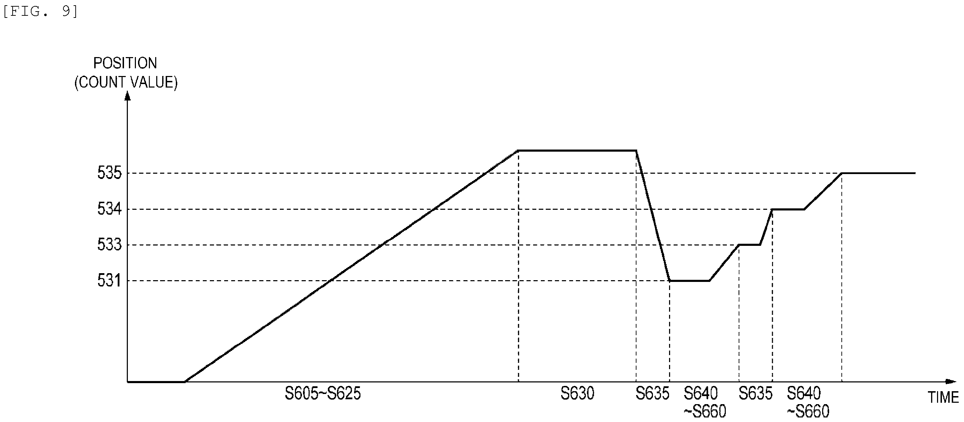

The display screen 500 shows an example of screen display in a case where the beginning position 531/the ending position 533 and the beginning position 534/the ending position 535 are designated, as described above. It can be also seen from the optical vascular axial sectional image 540 that measurement using the optical transceiver 372 is performed between the beginning position 531 and the ending position 533 and between the beginning position 534 and the ending position 535. FIG. 9 illustrates a relationship between lapse time and a count value (i.e., a position of the ultrasound transceiver 371 and the optical transceiver 372) in driving performed to obtain the measurement results displayed on the display screen 500.

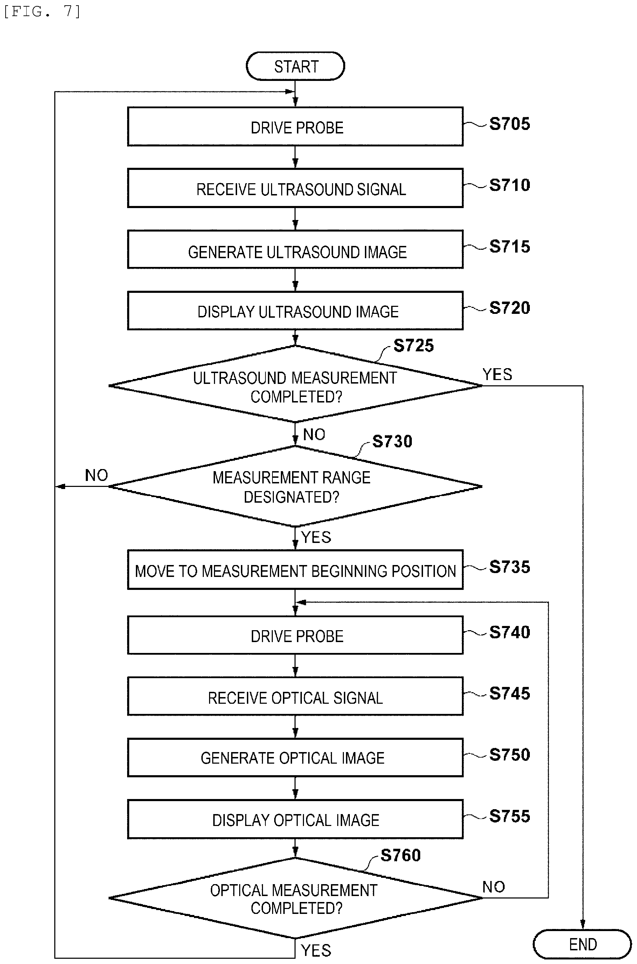

A method for displaying a vascular image is not limited to the method illustrated in FIG. 7. An example of a method for simultaneously displaying an optical image and an ultrasound image at the same intravascular position may include a method for simultaneously displaying an enlarged ultrasound vascular axial sectional image and an enlarged optical vascular axial sectional image at the same intravascular position.

According to this embodiment, driving for performing measurement using a first signal transceiver (e.g., the ultrasound transceiver 210) and driving for performing measurement using a second signal transceiver (e.g., the optical transceiver 230) are performed separately. In addition, an image obtained using the first signal transceiver and an image obtained using the second signal transceiver at the same intravascular position are simultaneously displayed. In this manner, according to this embodiment, since a user may compare images obtained using a plurality of signal transceivers at the same intravascular position, it is possible to support accurate diagnosis made by the user.

In addition, there are many cases where different signal transceivers have different optimal measurement conditions. For example, comparing the IVUS and OCT, there are many cases where rotation and movement are slower in measurement in the IVUS than in measurement in the OCT. With the configuration of this embodiment, since different signal transceivers may perform a scanning with different optimal conditions, it is possible to obtain a vascular image with better quality than a case where both of measurement using the first signal transceiver and measurement using the second signal transceiver are performed with one time driving.

In addition, particularly in a case of combination of IVUS and OCT, there is a case where an IVUS image is deteriorated due to an effect of a contrast agent or the like contained in a flush material used in a flush operation. According to this embodiment, by performing the IVUS measurement and the OCT measurement in separate drives, it is possible to prevent an image from being deteriorated due to the flush operation.

In addition, in the method of this embodiment, the IVUS measurement is first performed and the OCT measurement is then performed for a smaller measurement range. According to this method, since a range of the OCT measurement may be set to be small, it is possible to reduce the usage of the flush material.

### [Embodiment 2]

Next, a control device according to Embodiment 2 will be described. A control device 300 according to this embodiment has the same configuration as the control device 300 according to Embodiment 1 illustrated in FIG. 3. Hereinafter, the same configuration and process as in Embodiment 1 will not be repeated. In this embodiment, the reception part 340 can receive a designation of a measurement range using the optical transceiver 372 during driving of the probe 370 for measurement using the ultrasound transceiver 371. In this case, the drive control part 350 controls the drive part 360 to stop the driving for the measurement using the ultrasound transceiver 371. Then, the drive control part 350 controls the drive part 360 to drive the probe 370 for measurement using the optical transceiver 372. After completing the measurement using the optical transceiver 372, the drive control part 350 controls the drive part 360 to resume the driving for the measurement using the ultrasound transceiver 371 from a position before the driving stop.

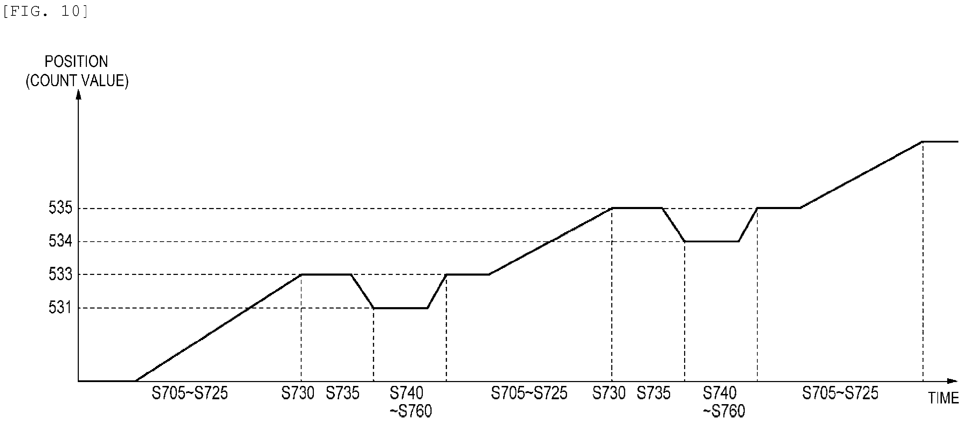

Hereinafter, a process in this embodiment will be described in detail. FIG. 7 is a flow chart of a process in this embodiment. FIG. 10 is a view illustrating a relationship between lapse time and motion of a probe part in this embodiment. At Steps S705 to S725, as in Steps S605 to S625, intravascular measurement using the ultrasound transceiver 371 is performed. In this embodiment, according to a position designation received by the reception part 340, the measurement using the ultrasound transceiver 371 may be stopped. In order to determine whether or not the position designation has been received, when it is determined at Step S725 that the measurement has not been completed, the process proceeds to Step S730.

At Step S730, the drive control part 350 determines whether or not the reception part 340 has received the designation of the measurement range. When it is determined that the designation of the measurement range has been received, the process proceeds to Step S735. When it is determined that the designation of the measurement range has not been received, the process returns to Step S705 in which the measurement using the ultrasound transceiver 371 continues.

In this embodiment, when a user pushes a button "Bookmark" through the operation panel 112, the designation of the measurement range is input. In one embodiment, a predetermined range including the position of the ultrasound transceiver 371 when the user pushes the button "Bookmark" is treated as the measurement range. For example, a measurement ending position may be the position of the ultrasound transceiver 371 and a measurement beginning position may be a position apart upward by a predetermined distance from the position of the ultrasound transceiver 371. A method for inputting the designation of the measurement range is not limited to the above-described method but may be any of different methods including the method described in Embodiment 1.

Through the process of Steps S735 to S760, the intravascular measurement using the optical transceiver 372 is performed for the measurement range designated at Step S730 . This process is the same as that of Steps S635 to S660 in Embodiment 1 and explanation of which will not be repeated.

After Step S760, the process of Steps S705 to S725 is performed to resume the measurement using the ultrasound transceiver 371 which has been stopped. Specifically, the ultrasound transceiver 371 is moved to the position when the measurement has been stopped, and, thereafter, the drive control part 350 controls the drive part 360 to resume the measurement. When the measurement ending position is the position of the ultrasound transceiver 371 when the designation of the measurement range is input, at the time of the end of the measurement using the optical transceiver 372, the ultrasound transceiver 371 stays substantially at the same as the position when the measurement has been stopped. Therefore, after the measurement using the optical transceiver 372 is ended, it is possible to resume the measurement using the ultrasound transceiver 371 without driving to move the ultrasound transceiver 371 at the measurement beginning position.

In the meantime, as in a case where a user directly designates the measurement ending position, there exists a case where the measurement ending position does not coincide with the position of the ultrasound transceiver 371 when the designation of the measurement range is input. In addition, in consideration of deviation between the position of the ultrasound transceiver 371 and the position of the optical transceiver 372 on the probe 370, a demand for exact alignment between the position of the ultrasound transceiver 371 when the measurement has been stopped and the position of the ultrasound transceiver 371 when the measurement is resumed may be considered. Further, in order to resume the measurement after the radial scanning is stabilized, it may be considered to resume the measurement from a position beyond the position of the ultrasound transceiver 371 when the measurement has been stopped. In these cases, the drive control part 350 controls the drive part 360 to resume the measurement using the ultrasound transceiver 371 after moving the ultrasound transceiver 371 to the measurement beginning position.

When it is determined at Step S725 that the measurement using the ultrasound transceiver 371 has been completed, the process of this embodiment is ended. Thereafter, setting of a region of interest and measurement of the region of interest using the optical transceiver 372 may be performed according to the same method as in Embodiment 1. In addition, according to an instruction from the user, the display control part 330 may control the display part 380 to simultaneously display an optical image and an ultrasound image at the same intravascular position.

### [Embodiment 3]

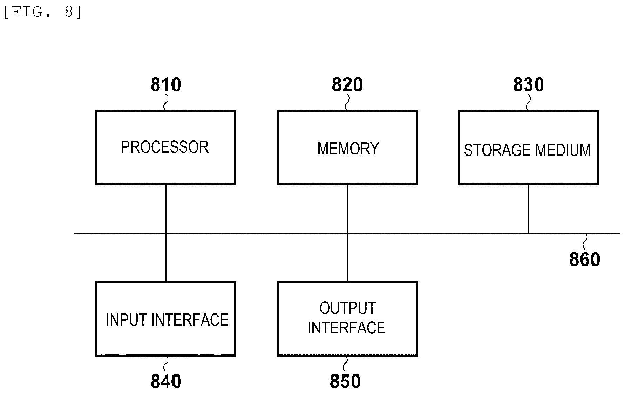

The functions of the various parts illustrated in FIG. 3 may be implemented with a general-purpose computer. FIG. 8 is a view illustrating the basic configuration of a computer. In FIG. 8, a processor 810 is, e.g., a CPU and controls the overall operation of the computer. A memory 820 is, e.g., a RAM and stores programs and data temporarily. A computer-readable storage medium 830 is, e.g. , a hard disk or a CD-ROM and stores programs and data in a non-transient manner. In this embodiment, programs which are stored in the storage medium 830 for implementing the functions of the various parts are read into the memory 820. Then, when the processor 810 executes the programs on the memory 820, the processes of the above-described steps are executed to implement the functions of the various parts.

In FIG. 8, an input interface 840 is an interface for acquiring information from an external device and is connected to, e.g., the operation panel 112 and so on. In addition, an output interface 850 is an interface for outputting information to an external device and is connected to, e.g., the LCD monitor 113 and so on. A bus 860 interconnects the above-described various parts for data exchange therebetween.

### [Other Embodiments]

Although the control device 300 of Embodiment 1 includes the drive control part 350 for driving the probe 370, the drive control part 350 may be replaced with other element or may be omitted. In addition, although the probe 370 of Embodiment 1 has both of the ultrasound transceiver 371 and the optical transceiver 372, these transceivers may be separately installed in separate probes.

For example, in one embodiment, the control device 300 acquires a vascular image obtained based on a signal obtained from the first signal transceiver on a probe inserted in the blood vessel during the first driving for the probe. In addition, the control device 300 acquires a vascular image obtained based on a signal obtained from the second signal transceiver on another probe during the second driving different from the first driving for the probe inserted in the blood vessel. The probe on which the first signal transceiver is installed and the another probe on which the second signal transceiver is installed may be same or different. In this case, the control device 300 acquires information indicating the position of the first signal transceiver when a signal is obtained, and information indicating the position of the second signal transceiver when a signal is obtained. Then, the control device 300 controls the display part 380 to simultaneously display a first vascular image at a first intravascular position, obtained using the first signal transceiver, and a second vascular image at a first intravascular position, obtained using the second signal transceiver.

With this configuration, vascular images at the same position based on signals obtained from different signal transceivers during different driving are automatically displayed side by side. Therefore, it is possible to perform a measurement with different optimal conditions for the respective signal transceivers. The images with high quality obtained thus are presented in an easily-comparable form, thereby allowing a user to make an easy diagnosis.

The following claims are appended to reveal the scope of the invention.

## Claims

1. A control device (300) comprising:
a drive control unit (350) configured to control a drive unit that drives a probe (101) inserted in a blood vessel to execute a first driving to perform a measurement using a first signal transceiver on the probe (101), said first signal transceiver being an intravascular ultrasound IVUS-transceiver, and a second driving to perform a measurement using a second signal transceiver on the probe (101), said second signal transceiver being an optical coherent tomography OCT-transceiver;
an acquisition unit (310) configured to (i) acquire a signal measured using the first signal transceiver on the probe (101) during the first driving, (ii) information indicating a position of the first signal transceiver when the signal is obtained, (iii) a signal measured using the second signal transceiver on the probe (101) during the second driving, and (iv) information indicating a position of the second signal transceiver when the signal is obtained; and
a display control unit (330) configured to control a display unit (380, 500) to simultaneously display a first vascular image at a first intravascular position obtained using the first signal transceiver and a second vascular image at the first intravascular position obtained using the second signal transceiver, **characterized in that** the first vascular image is an ultrasound cross-sectional image and the second vascular image is an optical cross-sectional image and said first
and second vascular images are generated from line data with which the information acquired by the acquisition unit indicates a measurement direction by the first and the second signal transceivers when the signal is obtained while the probe is being rotated, and are displayed such that rotational angles of the first vascular image and the second vascular image are adjusted such that their angular directions are aligned, the control device further comprising a generation unit (320) configured to generate a vascular axial sectional image of the blood vessel based on the signal from the first signal transceiver,
wherein the display control unit (330) further controls the display unit (380, 500) to display the vascular axial sectional image, and the control device further comprising a reception unit (340) configured to receive the designation of a measurement range in the vascular axial section image which is based on the signal obtained by the first signal transceiver during or after the first driving,
wherein the second signal transceiver performs the measurement within the designated measurement range during the second driving.

2. The control device (300) according to claim 1, wherein, when the reception unit (340) receives the designation when the first signal transceiver is at a second position during the first driving, the drive control unit (350) controls the drive unit to stop the first driving, execute the second driving, and then resume the first driving.

3. A diagnostic imaging system comprising:
a control device (300) according to claim 1; and
a drive unit configured to drive a probe (101) inserted in a blood vessel.

## Patentansprüche

1. Steuerungsvorrichtung (300), umfassend:
eine Antriebssteuerungseinheit (350), die so konfiguriert ist, dass sie eine Antriebseinheit steuert, die eine Sonde (101) antreibt, welche in ein Blutgefäß eingeführt ist, um ein erstes Antreiben auszuführen, um eine Messung unter Verwendung eines ersten Signal-Sender/Empfängers an der Sonde (101) durchzuführen, wobei der erste Signal-Sender/Empfänger ein intravaskulärer Ultraschall IVUS-Sender/Empfänger ist, und ein zweites Antreiben auszuführen, um eine Messung unter Verwendung eines zweiten SignalSender/Empfängers an der Sonde (101) durchzuführen, wobei der zweite Signal-Sender/Empfänger ein optischer Kohärenztomographie OCT-Sender/Empfänger ist;
eine Erfassungseinheit (310), die so konfiguriert ist, dass sie (i) ein Signal, das unter Verwendung des ersten Signal-Sender/Empfängers an der Sonde (101) während des ersten Antreibens gemessen wird, (ii) Informationen, die eine Position des ersten Signal-Sender/Empfängers anzeigen, wenn das Signal erhalten wird, (iii) ein Signal, das unter Verwendung des zweiten SignalSender/Empfängers an der Sonde (101) während des zweiten Antreibens gemessen wird, und (iv) Informationen, die eine Position des zweiten SignalSender/Empfängers anzeigen, wenn das Signal erhalten wird, erfasst; und
eine Anzeigesteuerungseinheit (330), die so konfiguriert ist, dass sie eine Anzeigeeinheit (380, 500) steuert, um gleichzeitig eine erste vaskuläre Abbildung an einer ersten intravaskulären Position, die unter Verwendung des ersten Signal-Sender/Empfängers erhalten wurde, und eine zweite vaskuläre Abbildung an der ersten intravaskulären Position, die unter Verwendung des zweiten Signal-Sender/Empfängers erhalten wurde, anzuzeigen, **dadurch gekennzeichnet, dass** die erste vaskuläre Abbildung ein Ultraschall-Querschnittsbild ist und die zweite vaskuläre Abbildung ein optisches Querschnittsbild ist und die erste und die zweite vaskuläre Abbildung aus Liniendaten erzeugt werden, mit denen die von der Erfassungseinheit erfasste Information eine Messrichtung durch den ersten und den zweiten Signal-Sender/Empfänger angibt, wenn das Signal erfasst wird, während die Sonde gedreht wird, und derart angezeigt werden, dass Drehwinkel der ersten vaskulären Abbildung und der zweiten vaskulären Abbildung derart eingestellt werden, dass ihre Winkelrichtungen ausgerichtet sind,
die Steuerungsvorrichtung ferner eine Erzeugungseinheit (320) umfasst, die so konfiguriert ist, dass sie ein vaskuläres axiales Schnittbild des Blutgefäßes auf der Grundlage des Signals von dem ersten SignalSender/Empfänger erzeugt, wobei die Anzeigesteuerungseinheit (330) ferner die Anzeigeeinheit (380, 500) steuert, um das vaskuläre axiale Schnittbild anzuzeigen, und die Steuerungsvorrichtung ferner eine Empfangseinheit (340) umfasst, die so konfiguriert ist, dass sie die Kennzeichnung eines Messbereichs in dem vaskulären axialen Schnittbild empfängt, die auf dem von dem ersten Signalsender-Empfänger während oder nach dem ersten Antreiben erhaltenen Signal basiert, wobei der zweite Signalsender-Empfänger die Messung innerhalb des gekennzeichneten Messbereichs während des zweiten Antreibens durchführt.

2. Steuerungsvorrichtung (300) nach Anspruch 1, wobei die Antriebssteuerungseinheit (350), wenn die Empfangseinheit (340) die Kennzeichnung empfängt, wenn sich der erste Signal-Sender/Empfänger während des ersten Antreibens an einer zweiten Position befindet, die Antriebseinheit steuert, um das erste Antreiben zu stoppen, das zweite Antreiben durchzuführen und danach das erste Antreiben wieder aufzunehmen.

3. Diagnostisches Bildgebungssystem, umfassend:
Steuerungsvorrichtung (300) nach Anspruch 1; und
eine Antriebseinheit, die konfiguriert ist, um eine in ein Blutgefäß eingeführte Sonde (101) anzutreiben.

## Revendications

1. Dispositif de commande (300) comprenant :
une unité de commande d'entraînement (350) configurée pour commander une unité d'entraînement qui entraîne une sonde (101) insérée dans un vaisseau sanguin pour exécuter un premier entraînement afin de réaliser une mesure à l'aide d'un premier émetteur-récepteur de signal sur la sonde (101), ledit premier émetteur-récepteur de signal étant un émetteur-récepteur d'échographie intravasculaire IVUS, et un deuxième entraînement afin de réaliser une mesure à l'aide d'un deuxième émetteur-récepteur de signal sur la sonde (101), ledit deuxième émetteur-récepteur de signal étant un émetteur-récepteur de tomographie en cohérence optique OCT ;
une unité d'acquisition (310) configurée pour acquérir (i) un signal mesuré à l'aide du premier émetteur-récepteur de signal sur la sonde (101) lors du premier entraînement, (ii) des informations indiquant une position du premier émetteur-récepteur de signal lorsque le signal est obtenu, (iii) un signal mesuré à l'aide du deuxième émetteur-récepteur de signal sur la sonde (101) lors du deuxième entraînement, et (iv) des informations indiquant une position du deuxième émetteur-récepteur de signal lorsque le signal est obtenu ; et
une unité de commande d'affichage (330) configurée pour commander une unité d'affichage (380, 500) pour afficher simultanément une première image vasculaire à une première position intravasculaire obtenue à l'aide du premier émetteur-récepteur de signal et une deuxième image vasculaire à la première position intravasculaire obtenue à l'aide du deuxième émetteur-récepteur de signal, **caractérisé en ce que** la première image vasculaire est une image en coupe transversale échographique et la deuxième image vasculaire est une image en coupe transversale optique et lesdites première et deuxième images vasculaires sont générées à partir de données de ligne avec lesquelles les informations acquises par l'unité d'acquisition indiquent une direction de mesure par les premier et deuxième émetteurs-récepteurs de signal lorsque le signal est obtenu alors que la sonde est tournée, et sont affichées de telle sorte que des angles de rotation de la première image vasculaire et de la deuxième image vasculaire sont ajustés de telle sorte que leurs directions angulaires sont alignées, le dispositif de commande comprenant en outre une unité de génération (320) configurée pour générer une image en coupe axiale vasculaire du vaisseau sanguin sur la base du signal provenant du premier émetteur-récepteur de signal,
dans lequel l'unité de commande d'affichage (330) commande en outre l'unité d'affichage (380, 500) pour afficher l'image en coupe axiale vasculaire, et le dispositif de commande comprenant en outre une unité de réception (340) configurée pour recevoir la désignation d'une plage de mesure dans l'image en coupe axiale vasculaire qui est basée sur le signal obtenu par le premier émetteur-récepteur de signal pendant ou après le premier entraînement,
dans lequel le deuxième émetteur-récepteur de signal réalise la mesure dans la plage de mesure désignée lors du deuxième entraînement.

2. Dispositif de commande (300) selon la revendication 1, dans lequel, lorsque l'unité de réception (340) reçoit la désignation lorsque le premier émetteur-récepteur de signal se trouve dans une deuxième position pendant le premier entraînement, l'unité de commande d'entraînement (350) commande à l'unité d'entraînement d'arrêter le premier entraînement, d'exécuter le deuxième entraînement, puis de reprendre le premier entraînement.

3. Système d'imagerie diagnostique comprenant :
un dispositif de commande (300) selon la revendication 1 ; et
une unité d'entraînement configurée pour entraîner une sonde (101) insérée dans un vaisseau sanguin.
